# EUROPEAN PATENT APPLICATION

(11) **EP 2 468 709 A1**
(43) Date of publication of application: **27.06.2012**
(21) Application number: 10193120.2
(22) Date of filing: 30.11.2010
(51) Int. Cl.: C07C 45/52, C07C 47/22

(54) **A process for producing acrolein**

(71) Applicant: BioFuel-Solution AB, 200 61 Limhamn (SE)
(72) Inventor: Hulteberg, Christian, SE-217 46, Malmö (SE); Brandin, Jan, SE-215 79, Malmö (SE)
(74) Representative: Ström & Gulliksson AB

(57) **Abstract**

Disclosed is a process for dehydrating glycerol into acrolein over an acidic catalyst in gas phase in the presence of hydrogen, minimizing side reactions forming carbon deposits on the catalyst.

## Description

### Field of the invention

The present invention relates to a process for producing acrolein. More specifically it relates to a process for dehydrating glycerol into acrolein over an acidic catalyst in gas phase, minimizing side reactions forming carbon deposits on the catalyst.

### Background

There is a growing interest and need for renewable fuels throughout the world to replace fossil fuels. Furthermore, there is also a need for hydrocarbons containing activated groups, such as carbon-carbon and carbon-oxygen double bonds. Such unsaturated hydrocarbons, not originating from petroleum, could also find use as monomers within the polymer industry and as starting material from preparing other chemicals of interest.

Glycerol or glycerin, C₃H₈O₃, is a viscous uncolored transparent liquid in pure state at ambient conditions. Glycerol has traditionally been a byproduct from soap manufacturing, using animal and vegetable oils and fats. Today the majority of the produced glycerol originates from the production of biodiesel, such as methyl esters of fatty acids, by trans-esterfication of the same types of triglycerides with an alcohol, for instance methanol. The amount of glycerol produced is approximately 1:9 by weight of the biodiesel produced.

Due to the growing interest in renewable fuels for transportation, the production of biodiesel has increased tremendously over the last 10 years and as a result, the glycerol market has been saturated and the value of glycerol has decreased radically. Because of the abundance and low cost, the interest in glycerol as a fuel has grown. The glycerol has a heating value (heat of combustion) of 16 MJ/kg, but is due to the high flame point (180°C) difficult to bum. Thereby use of glycerol as a fuel requires support fuel and specially designed burners.

Further, there have also been interest in using glycerol as starting material for producing other chemicals such as ethane and propane (cf. WO2010/052208), and acrylic acid (cf. WO2006/114506).

In its natural state, acrolein is a liquid but with a low boiling point, i.e. 53°C. The low boiling point makes it difficult to handle and transport. Thus, a feasible process for dehydrating glycerol to acrolein, wherein the produced acrolein may be used directly and produced continuously would be highly desirable.

The production of acrolein from glycerol has been known for several decades in the gas phase using acidic catalysts. In 1918, Sabatier and Gaudion (P. Sabatier, G. Gaudion, Compt. Rend. 166 (1918) 1033) produced 10% acrolein yield from glycerol by gas-phase dehydration over Al₂O₃ or UO₂ catalysts at temperatures of 360 and 350 °C, respectively, with byproducts being ethanol, water, and propenol as other products. In 1928, Freund stated that acrolein could be produced from glycerol at 180 °C using silica (US 1,672,378). Twenty years later, Hoyt and Manninen reported a method to produce acrolein from glycerol using solid phosphoric acid catalysts (US 2,558,520). Heinemann et al. studied the dehydration of glycerol with activated bauxite catalysts and showed acrolein yields of up to 42% from glycerol working in gas phase at 430 °C (H. Heinemann, R.W. Wert, Ind. Eng. Chem. 41 (1949) 2928).

The major issue in producing the acrolein is the formation of solid carbon, i.e. carbon deposits, which inevitably will block the catalyst system used and eventually creates process upsets (Journal of Catalysis 269 (2010) 71-79). The carbon formation is believed to be a result of the formation of polymers of glycerol on the catalyst surface, which are then pyrolysed to form solid carbon residues (K. Pathak et al. Applied Catalysis A: General 372 (2010) 224-238). Additionally, the formation of hydroxyacetone is present in low amounts lower the overall yield of acrolein (Microporous and Mesoporous Materials 131 (2010) 28-36).

Thus, it would be highly desirable to prevent the formation of solid carbon and thereby allow for continuous production of acrolein and eliminate the need to regenerate the catalyst repeatedly.

To deal with the issue of carbon formation there has been a number of suggestions. In WO 2006/087083 a method of continuous addition of oxygen for the in-situ oxidation of the carbon is described. A different approach is described in WO 2009/156664, in which a small amount of inorganic acid is added to the reaction mixture to keep the catalyst in the acid state. Other suggestions also deal with moving bed reactors where a portion of the catalyst can be regenerated using air US 2008/183019.

However, the addition of oxygen or air inevitably lowers the potential overall yield of the process, as well as creates a potential safety hazard. This safety hazard is avoided to some extent by using a moving bed with separate regeneration; this however requires much more elaborate technical solutions to the problem. The addition of acids to the reactor also adds complexity in the operation as well as creates a potential corrosion issue, requiring more exclusive materials to be used in the construction of the equipment.

Thus, there is need within the art for an alternative method of producing acrolein from glycerol, overcoming the above-mentioned deficiencies.

### Summary

Consequently, the present invention seeks to mitigate, alleviate, eliminate or circumvent one or more of the above-identified deficiencies in the art and disadvantages singly or in any combination by providing a method of producing acrolein from glycerol comprising the steps of:
- passing a mixed stream of gaseous water, glycerol and hydrogen over an acidic catalyst, catalyzing the dehydration of glycerol to acrolein, such as WO₃*H₂O supported on ZrO₂,
wherein the water and the glycerol are present in the mixed stream in a molar ratio of 30:1 to 4:1, such as 20:1 to 5:1, the glycerol and the hydrogen are present in the mixed stream in a molar ratio of 6:1 to 1:18, the temperature over said acidic catalyst is from 200 to 450°C, such as 250 to 350°C, and the pressure over said acidic catalyst is 0.1 to 10 MPa, such as 0.1 to 2 MPa or 0.1 to 1 MPa.

The presence of hydrogen has been found to minimize carbon deposits on the catalyst. Further, the amount of hydrogen in the mixed stream may be two times the molar amount of glycerol or less, such as equal to or less than the molar amount of glycerol. At least part of hydrogen gas, present in the effluent of the reactor, may be separated from other gases in the effluent of the reactor and subsequently fed to the reactor once more. Further, at least part of the effluent of the reactor, comprising acrolein, may be condensed, and acrolein, present in the condensate, subsequently is separated, such as vaporized, from the condensate.

Another aspect of the invention relates to a method of producing acrylic acid from glycerol. In such a method aqueous glycerol may be dehydrated to acrolein in the presence of hydrogen, as described above. The method further comprises the steps of:
- separating hydrogen from other gases present in the effluent of the reactor, in which glycerol is dehydrated to acrolein, by condensing water and acrolein;
- optionally feeding the separated hydrogen to the reactor once more;
- separating acrolein from the condensate, comprising water and acrolein, by vaporization of it;
- mixing the vaporized acrolein with oxygen; and
- passing the mixed gaseous stream, comprising acrolein and oxygen, over a catalyst, catalyzing the oxidation of acrolein to acrylic acid, at a temperature of 200 to 400°C, to obtain acrylic acid. The catalyst, catalyzing the oxidation of acrolein to acrylic acid, may be a catalyst comprising molybdenum and vanadium oxides and at least one further component selected from the group consisting of tungsten, copper, tin, antimony, niobium, nickel, cobalt, tellurium, bismuth, iron, lead, zinc, silicon, titanium, zirconium, alkaline metals and oxides thereof.

Another aspect of the invention relates to use of hydrogen to prevent formation of carbon deposits on an acidic catalyst, catalyzing the dehydration of glycerol to acrolein, in the dehydration of glycerol, in gas phase and in the presence of water, to acrolein.

Further advantageous features of the invention are defined in the dependent claims. In addition, advantageous features of the invention are elaborated in embodiments disclosed herein.

### Description

### Brief description of the drawings:

Fig. 1 depicts an illustrative embodiment for producing acrolein from glycerol in the presence of hydrogen.
Fig. 2 depicts an illustrative embodiment for producing acrylic acid from glycerol in the presence of hydrogen, wherein acrolein is an intermediate.
Fig. 3 depicts the experimental set-up employed in example 1 and 2.
Fig. 4 depicts the yield of acrolein and side-products (CO, CO₂, hydroyacetone) in when nitrogen is used to lower the partial pressure of glycerol.
Fig. 5 depicts the yield of acrolein and side-products (CO, CO₂, hydroyacetone, propanal) in when hydrogen is used to lower the partial pressure of glycerol.

### Embodiments:

As described above, the production of acrolein from glycerol has been known for long in the gas phase using acidic catalysts, such as Al₂O₃, UO₂, silica, solid phosphoric acid catalysts, or activated bauxite catalysts. Further, the problems with carbon deposits are also well recognized in the art.

It has unexpectedly been found that the presence of hydrogen gas suppresses side-product formation, and especially the formation of carbon deposits, in dehydration of glycerol to acrolein over an acidic catalyst in gas phase.

An embodiment thus relates to the use of hydrogen to prevent formation of carbon deposits on an acidic catalyst, catalyzing the dehydration of glycerol to acrolein, in the dehydration of glycerol, in gas phase and in the presence of water, to acrolein.

Similarly, another embodiment relates to method for producing acrolein from glycerol in the presence of hydrogen in a reactor. Such a method comprises the step of:
- passing a mixed stream of gaseous water, glycerol and hydrogen over an acidic catalyst, catalyzing the dehydration of glycerol to acrolein, wherein
- the water and the glycerol may be present in the mixed stream in a molar ratio of 30:1 to 4:1, such as 20:1 to 5:1;
- the glycerol and the hydrogen may be present in the mixed stream in a molar ratio of 6:1 to 1:18, such as 3:1 to 1:3;
- the temperature over said acidic catalyst may be from 200 to 450°C, such as 250 to 350°C; and
- the pressure over said acidic catalyst may be 0.1 to 10 MPa, such as 0.1 to 2 MPa or 0.1 to 1 MPa.

The presence of hydrogen in the reaction mixture has further been found, not only to counteract the formation of carbon deposits, but also to lower the formation of hydroxyacetone. Experiments, in which hydrogen was replaced by nitrogen, did indicate that these effects may be attributed to the presence of hydrogen and is not merely an effect of lowering the partial pressure of glycerol.

Typically, the mixed stream may comprise 45 to 95 vol% steam, 3 to 11 vol % glycerol, and 2 to 54 vol% hydrogen. Although, the mixed stream in principal may comprise other components than steam, glycerol, and hydrogen, such as nitrogen, it is preferred if these components, i.e. steam, glycerol, and hydrogen, constitute at least 95 vol%, such as at least 97.5, 99 or 99.5 vol%, of the mixed stream.

None, or a very small amount, hydrogen is normally consumed over the acidic catalyst, thus the effect is not related to consumption of hydrogen. Instead, it may be, without being bond to any theory, that the hydrogen blocks the active sites on the catalyst propagating glycerol condensation reactions leading to carbon deposits. The presence of hydrogen also seems to affect the active sites for producing the hydroxyacetone side-product to lower the reaction rate for this particular reaction.

As indicated above, the molar ratio of glycerol and hydrogen present in the mixed stream may be 6:1 to 1:18, such as 3:1 to 1:3. The amount of hydrogen present in the mixed stream may preferably be sufficient to prevent formation of carbon deposits. Further, of process economical reasons it is preferred to keep the amount as low as possible. Thus, the molar amount of hydrogen in the mixed stream may be two times the molar amount of glycerol or less, such as equal the molar amount of glycerol or less than the molar amount of glycerol.

If acrolein is to react with hydrogen in a subsequent reaction, the molar ratio of glycerol and hydrogen present in the mixed stream may be 1:1 or lower, such as 1:2 or 1:5 or lower. Using a 1:1 ratio may imply that the need to separate and re-cycle hydrogen may be dispensed with, as the hydrogen will be consumed in the subsequent reaction.

The acidic catalyst may typically be present in a reaction vessel, i.e. a reactor, in which the dehydration of glycerol to acrolein takes place. Such a reactor may have at least one inlet for introducing water, glycerol, and/or hydrogen. In addition, the reactor may have at least one outlet for the effluent comprising acrolein and water.

Further, the catalyst used may have the shape of pellets, monoliths or any other form relevant to the situation.

The reactor in which the dehydration takes place may be operated in an adiabatic mode, or heat may be withdrawn or supplied along side or inside the reactor. Further, the reactor, wherein the acidic catalyst is present, may be a fixed bed reactor. Reactions, such as dehydrations of glycerol to acrolein, which are known to give rise to carbon deposits, are normally not performed in fixed bed reactor due to the difficulty of re-generating the catalyst within the reactor. Thus, fluidized beds or moving beds are typically used in systems wherein the catalyst is to be repeatedly re-generated.

According to an embodiment, glycerol and water are mixed and the resulting mixture is vaporized before being introduced into the reactor. Hydrogen may be mixed into the water/glycerol mixture before vaporization. It may also be used as a sweep-gas during vaporization. Hydrogen may even be introduced to the reactor separate from glycerol and/or water.

Preferably, the water, the glycerol, and/or the hydrogen are heated before being fed to the reactor. The temperature within the reactor may be higher than the temperature of the feed(s).

According to an embodiment the temperature within the reactor may be from 200 to 450°C, such as 250 to 350°C. Further, the temperature of mixed stream of glycerol, steam and hydrogen fed to the reactor may be 200 to 450°C, such as 250 to 350°C.

In WO 2006/087084 and US 5,387,720, which hereby is incorporated in their entirety by reference, several aspects of the conversion of glycerol in gas phase to acrolein are disclosed. Furthermore, several examples of suitable catalysts are disclosed.

According to an embodiment, examples of catalysts catalyzing dehydration reactions, such as the dehydration of glycerol to form acrolein, may be chosen from natural or synthetic siliceous materials or acidic zeolites; mineral supports, such as oxides, coated with mono-, di-, tri- or polyacidic inorganic acids; oxides or mixed oxides, or alternatively heteropolyacids. Further, the catalyst may be chosen from zeolites, Nafion^{®} composites (based on sulfonic acid of fluorinated polymers), chlorinated aluminas, phospho- tungstic and/or silicotungstic acids and acid salts, and various solids of metal oxide type such as tantalum oxide Ta₂O₅, niobium oxide Nb₂O₅, alumina Al₂O₃, titanium oxide TiO₂, zirconia ZrO₂, tin oxide SnO₂, silica SiO₂ or silico-aluminate SiO₂-Al₂Oa, impregnated with acidic functions such as borate BO₃, sulfate SO₄, tungstate WO₃, phosphate PO₄, silicate SiO₂ or molybdate MoO₃.

According to an embodiment the catalyst comprises an acidic active phase, such as a mineral acid, a solid acid, an acidic zeolite or an acidic metal oxide:

According to an embodiment the active phase of the catalyst comprises a mineral acid, such as H₃PO₄, H₂SO₄, H₃BO₃. The active phase may also comprise a solid acid, such as alumina (Al₂O₃), titanium dioxide (TiO₂). Furthermore, the solid acid may be an acidic zeolite, i.e. the H-form of zeolites, such as H-mordenite, H-Beta, H-ZSM-5 etc) or an acidic transition metal oxide, such as molybdic acid (MoO₃*H₂O), tungstic acid (WO₃*H₂O), and vanadic acid (H₃VO₄).

The active phase may be mounted or deposited on a carrier material. If the active phase comprises a mineral acid, then mounting of the active phase on the carrier material is to be preferred. If the active phase comprises a metal oxide then deposition of the active phase on the carrier material is to be preferred. The carrier material may comprise or even consist of silica (SiO₄), Alumina (Al₂O₃), carbon or ZrO₂.

According to an embodiment, the catalyst comprises or even consist of WO₃*H₂O supported on ZrO₂. WO₃ on ZrO₂ has been shown to be efficient in producing acrolein from glycerol.

Further, the catalyst may also be selected from the group consisting of catalysts comprising iron phosphorus oxides and at least one promoter selected from the group consisting of alkali metals, alkaline-earth metals, Al, Si, B, Co, Cr, Ni, V, Zn, Zr, Sn, Sb, Ag, Cu, Nb, Mo, Y, Mn, Pt, Rh and the rare earths La, Ce, Sm, catalysts comprising ammonium tungsten, tungsten acid or tungsten oxide, at least one promoter selected from the group consisting of gold, silver, copper, vanadium, platinum, palladium, ruthenium, samarium, cerium, yttrium, scandium, lanthanum, zinc, magnesium, cobalt, nickel, and optionally lithium, sodium, potassium and/or cesium, and heteropolyacidic catalysts, such as silicotungstic acid, phosphotungstic acid and phosphomolybdic acid supported on alumina, titania or zirkonia.

The effluent of the reactor comprising acrolein, and typically also steam and hydrogen, may be treated in various ways. Typically, steam present in the effluent may be separated from the other constituents of the effluent. According to an embodiment, the effluent may be cooled to condense at least water or at least water and acrolein. If water and acrolein are condensed, hydrogen may subsequently be separated from the condensed water/glycerol mixture, such as by flashing. Further, acrolein may subsequently be separated from the condensed water/glycerol mixture, such as by flashing. The effluent may also be subject to further reaction step(s), before side products and/or steam is separated from the product.

According to an embodiment, the effluent of the reactor comprising acrolein may be passed through a second catalytic step in which the acrolein is reacted with oxygen to yield acrylic acid. Before oxidizing acrolein, hydrogen may be separated from the gaseous effluent of the reactor comprising acrolein and hydrogen. Further, acrolein may typically be separated fully or in part from water before being oxidized. Various catalysts may be used to catalyze the oxidation of acrolein. The oxidation is preferably performed in gas-phase. Due to the different boiling points, acrolein is easily separated from water by destilation.

Accordingly, an embodiment relates to a method of producing acrylic acid from glycerol, comprising the steps of:
- dehydrating aqueous glycerol to acrolein as disclosed herein;
- separating hydrogen from other gases present in the effluent of the reactor by condensation of water and acrolein;
- optionally feeding the separated hydrogen to the reactor, wherein glycerol is dehydrated to acrolein, once more;
- separating acrolein from the condensate, comprising water and acrolein, by vaporization of it;
- mixing the vaporized acrolein with oxygen; and
- passing the mixed gaseous stream, comprising acrolein and oxygen, over a catalyst, catalyzing the oxidation of acrolein to acrylic acid, at a temperature of 200 to 400°C to obtain acrylic acid.

Evidently, various aspect of the method for producing acrolein from glycerol disclosed herein in the presence of hydrogen, are applicable also to the method of producing acrylic acid from glycerol, wherein acrolein to be oxidized is obtained from glycerol in the presence of hydrogen.

Non-limiting examples of catalysts, catalyzing the oxidation of acrolein to acrylic acid, are catalysts comprising molybdenum and vanadium oxides and at least one further component selected from the group consisting of tungsten, copper, tin, antimony, niobium, nickel, cobalt, tellurium, bismuth, iron, lead, zinc, silicon, titanium, zirconium alkaline metals and oxides thereof. US 2010/0121007 is devoted to catalysts for producing acrylic acid.

As already disclosed herein above, hydrogen is not consumed in the dehydration of glycerol to acrolein over the acidic catalyst. According to an embodiment, at least part of hydrogen gas in the effluent may be separated from other gases in the effluent of the reactor. The separated hydrogen may subsequently be fed once more to the reactor. Thus, the hydrogen may be recycled.

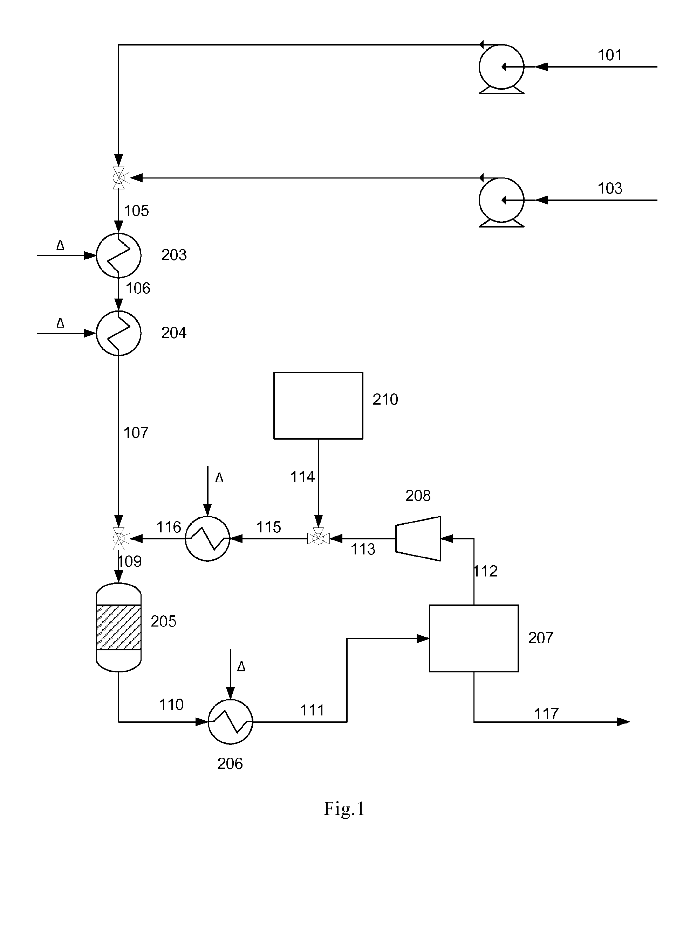

In Fig. 1, depicting an illustrative embodiment for producing acrolein from aqueous glycerol in gas phase, a schematic overview of an illustrative process is given. In the depicted process, two pumps supply the reaction pressure by compressing a water stream 101 and a glycerol stream 103. These streams 101, 103 may be combined to a water/glycerol stream 105 and vaporized in a first heater 203 to provide a gaseous water/glycerol stream 106, which may be superheated in a second heater 204 to provide a heated gaseous water/glycerol stream 107. The heated gaseous water/glycerol stream 107 may be mixed with a first hydrogen stream 116 and the resulting gaseous mixed stream 109 may be fed to a catalytic reactor 205 comprising the acidic catalyst, catalyzing the dehydration of glycerol to acrolein. The effluent 110 of the reactor, comprising acrolein, may be led to a cooler 206, which may cool the mixture to about 25°C or lower, to condensate acrolein, water and other high-boiling compounds, but not hydrogen. The resulting partially condensed stream 111 may be led to a flash vessel 207, separating a second hydrogen stream 112 from condensed stream 117 comprising acrolein. The second hydrogen stream 112 may contain small amounts of acrolein and water. To further increase the product yield and lower the compression work, a second chiller may thus be used, cooling the second hydrogen stream 112 further. The second hydrogen stream 112 separated from the effluent 110 may then be compressed in a compressor 208 and the resulting compressed second hydrogen stream 113 may optionally be mixed with a make-up hydrogen stream 114 from a hydrogen supply 210, to compensate for any loss of hydrogen. The resulting third hydrogen stream 115 may then be heated to provide the first stream hydrogen 116, which is mixed with the heated gaseous water/glycerol stream 107.

As disclosed herein, the first hydrogen stream 116 may also be mixed with the water/glycerol stream 105 or the gaseous water/glycerol stream 106. Further, the first hydrogen stream 116 may even be mixed into the water stream 101 or the glycerol stream 103.

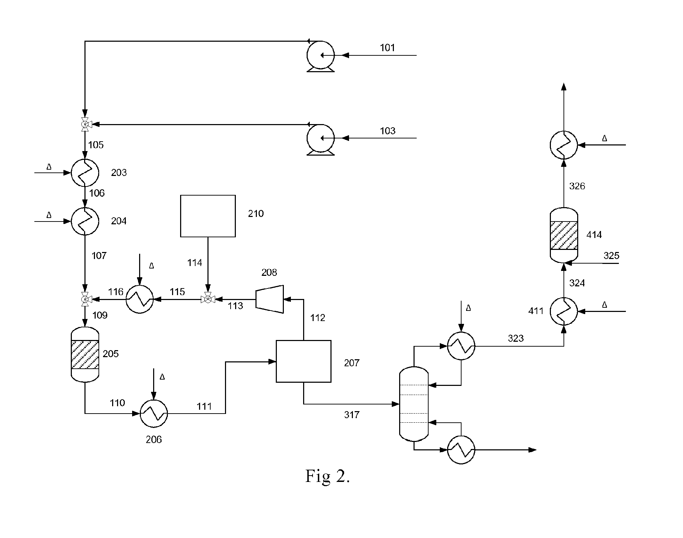

In Fig. 2, depicting an illustrative embodiment for producing acrylic acid from aqueous glycerol in gas phase, a schematic overview of an illustrative process is given. The condensed stream 117 comprising acrolein in Fig. 1 corresponds to stream 317 in Fig. 2.

Water and acrolein present in the condensed stream 317 may be separated by distillation to obtain a gaseous acrolein stream 323 and a liquid aqueous stream 320. The gaseous acrolein stream 323 may be heated in a heater 411. The heated gaseous acrolein stream 324 may be mixed with air 325 and passed over a catalyst 414, catalyzing the oxidation of acrolein to acrylic acid, to obtain a stream comprising acrylic acid 326.

As indicated above, an embodiment relates to the use of hydrogen to prevent formation of carbon deposits on an acidic catalyst, catalyzing the dehydration of glycerol to acrolein, in the dehydration of glycerol, in gas phase and in the presence of water, to acrolein. Evidently, such use may be in a process for producing acrolein from glycerol as disclosed herein. Thus, a further embodiment relates to the use of hydrogen to prevent formation of carbon deposits on an acidic catalyst in a process for producing acrolein from glycerol, as disclosed herein. Similarly, such use may also be in a process for producing acrylic acid from glycerol, wherein acrolein is an intermediate, as disclosed herein.

Although the present invention has been described above with reference to specific embodiments, it is not intended to be limited to the specific form set forth herein. Rather, the invention is limited only by the accompanying claims and, other embodiments than the specific above are equally possible within the scope of these appended claims, e.g. different than those described above.

In the claims, the term "comprises/comprising" does not exclude the presence of other elements or steps. Additionally, although individual features may be included in different claims, these may possibly advantageously be combined, and the inclusion in different claims does not imply that a combination of features is not feasible and/or advantageous.

In addition, singular references do not exclude a plurality. The terms "a", "an", "first", "second" etc do not preclude a plurality.

### Examples

| *Abbreviations* | |
|---|---|
| FID | Flame Ionization Detector |
| GC | Gas Chromatograph |
| TCD | Thermal Conductivity Detection |

The examples given below are only intended to further illustrate the invention and are by no means intended to limit the scope of the invention as defined by the appended claims.

### General

The feed in all the examples below consisted of an aqueous solution of 20 wt% glycerol. The solution was stored in a pressurized vessel and the inlet flow rate was controlled by a mass flow controller. The inlet liquid flow was heated and vaporized in a pre-heater. At this point, nitrogen or hydrogen gas could be introduced into the stream to facilitate the vaporization.

The reaction mixture was then introduced into a micro-reactor containing the acidic catalyst used in the experiments, 10 % WO₃/ZrO₂. The reactor system was fitted with automated process control, allowing for exact pressure and temperature control, as well as allowing for around the clock operation.

After leaving the reactor, the gas was cooled down and condensed in a water-cooled condenser. Thereafter a liquid sample was collected for analysis. The uncondensable gas was measured by a digital gas flow meter and a gas sample was collected in sample bags.

The liquid analysis was performed using a Varian CP-3800 gas chromatograph equipped with an 1177 split/split-less injector, a CP-wax 58/FFAP column and a flame ionization detector. The permanent gas analysis was performed using a Varian CP-4900 gas chromatograph, using both a mol-sieve 5A PLOT column and a PoraPlot column with two TCD detectors.

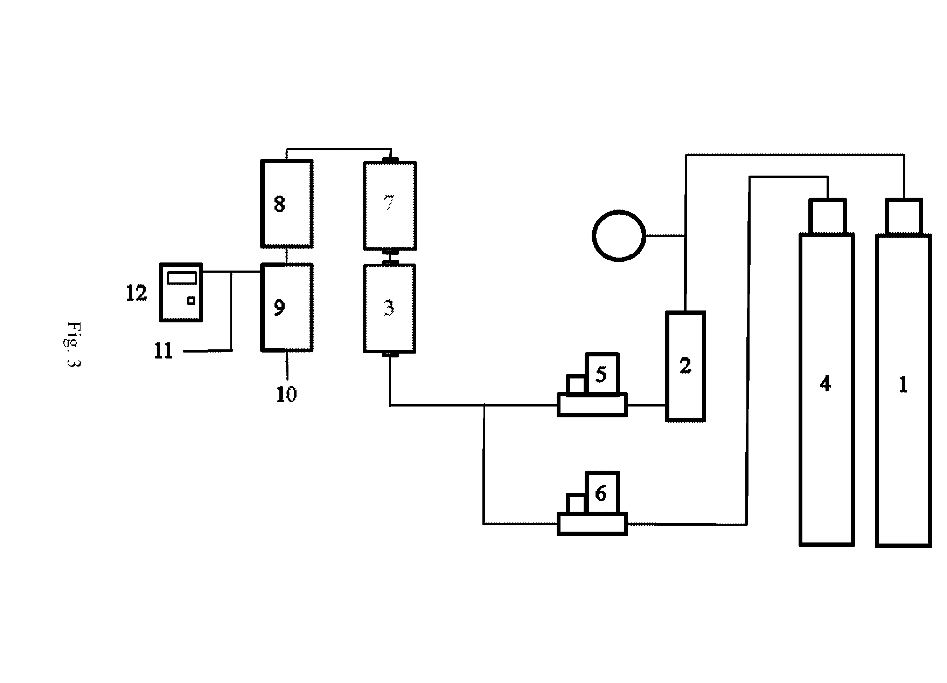

An outline of the experiment set-up is depicted in Fig. 3. The set-up comprises a nitrogen reservoir 1 connected to a pressurized vessel 2 comprising the glycerol/water mixture or the acrolein/water mixture to be fed to a pre-heater 3. A hydrogen or nitrogen reservoir 4 is also connected to the pre-heater 3. Both the connection of the hydrogen/nitrogen reservoir 4 and the pressurized vessel 2 are controlled by mass flow controllers 5 and 6. The pre-heater 3 is connected to a first reactor 7 which is connected to condenser 8, dividing the outlet flow of the reactors into a liquid stream and gas stream. The liquid and gas streams are separated in a vessel 9 into a liquid stream 10 and a gas stream 11 The flow of the gas stream may be measured may be measured by a gas flow meter 12.

The pre-heater 3 had a length of 250 mm and diameter of 25 mm and was filled with sintered alumina heat transfer media.

The reactor 7, being a tube-reactor had a length of 250 mm and diameter of 25 mm.

The preheater 3 and the reactors 7 were equipped with thermocouples at their in- and out-lets.

### Example 1 - production of acrolein with nitrogen to lower the partial pressure

Glycerol was converted to acrolein over a catalyst bed with a layer of catalyst with nitrogen used to lower the partial pressure of the reactants.

A catalyst layer consisting of 56 g of a catalyst, 10 w% of WO₃ supported on ZrO₂ in grains of the size 20-30 mesh, was used.

The inlet liquid stream consisted of 20 wt% of glycerol in water fed to the pre-heater 3 at 0.3 g/min. A gas stream of 100 ml/min of nitrogen was also fed to the pre-heater 3. The liquid stream was preheated and vaporized, to 280 °C, prior entering the first reactor 7. The inlet of reactor 1 was held at 300 °C and a pressure of 5 bar gauge was applied over reactor 7. The outlet stream was cooled down in the condenser 8 and the water was condensed. The liquid stream 10 was collected in a sample vessel, while the gas stream 11 was collected in a Tedlar gas bag. The liquid sample was analyzed with a GC equipped with FID and a WAX-column for hydrocarbons (propanol, propanal, propanoic acid etc.). The gas sample was analyzed with a two channel GC equipped with TCD for analyzing CO, CO₂, ethene, ethane etc.

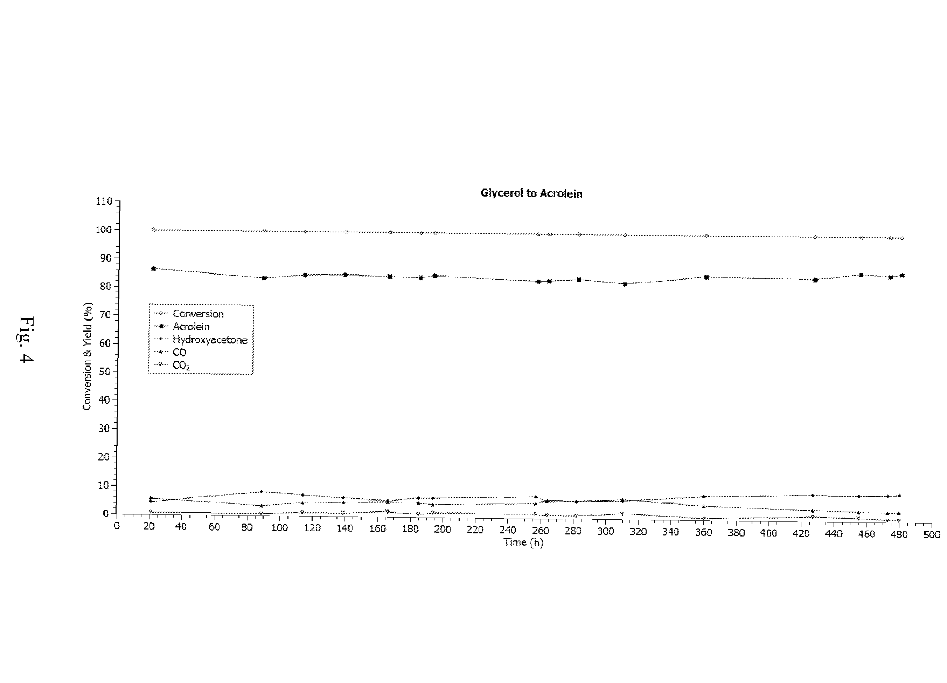

In Fig. 4 the result of a four hundred and eighty hour run is shown.

As seen, all glycerol was converted to below the limit of detection. Further, the yield of acrolein was above 80 %, with a side-production of 8% hydroxyacetone and small amounts of CO and CO₂.

However, during the 480 hour run, there was a steady increase in the pressure drop over the catalyst bed. Finally the experiment could not be continued and was terminated. Upon inspection it was showed that between 2 and 3 % of the total amount of carbon entered into the reactor had precipitated and accumulated in the catalyst bed and in the dead-space after the catalyst bed.

### Example 2 - production of acrolein with hydrogen to lower the partial pressure

In this example the same conditions and catalysts as in example 1 with the exception that hydrogen (100 ml/min) was used instead of nitrogen.

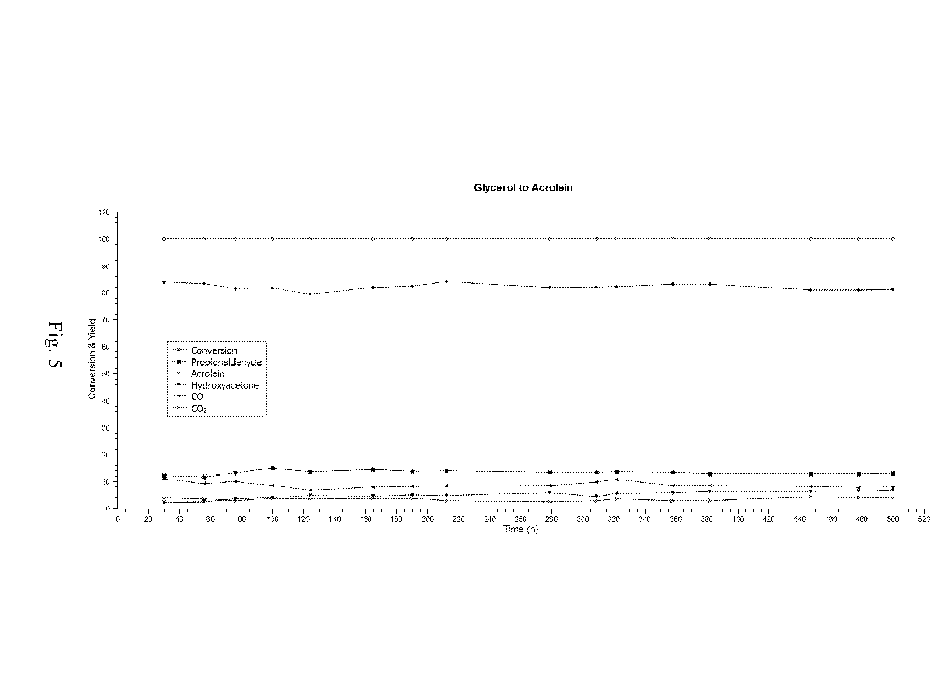

In Fig. 5 the yield of acrolein, CO₂, CO, hydroxyacetone and propionaldehyde are shown.

Again, glycerol was converted to beyond the detection limit and acrolein was yielded in amounts above 80%. The production of hydroxyacetone was lowered to 5% while the yields of CO and CO₂ essentially the same. Further, some 10% propionaldehyde was formed as a side product.

However, the largest and most important difference compared to example 1 is that there was no marked increase in pressure drop over the catalyst bed during the five hundred hours of testing, indicating very low amounts of carbon formation, if any. Thus, example 2 confirms that presence of hydrogen in the fed to the rector wherein glycerol is dehydrated to acrolein over an acidic catalyst may prevent formation of carbon deposits on the catalyst.

## Claims

1. A method for producing acrolein from glycerol in a reactor, comprising the step of:
- passing a mixed stream of gaseous water, glycerol and hydrogen over an acidic catalyst, catalyzing the dehydration of glycerol to acrolein,
wherein the water and the glycerol are present in the mixed stream in a molar ratio of 30:1 to 4:1, the glycerol and the hydrogen are present in the mixed stream in a molar ratio of 6:1 to 1:18, the temperature over said acidic catalyst is from 200 to 450°C, and the pressure over said acidic catalyst is 0.1 to 10 MPa.

2. The method according to claim 1, wherein the catalyst is WO₃*H₂O supported on ZrO₂.

3. The method according to any one of the preceding claims, wherein the reactor is a fixed bed reactor.

4. The method according to any one of the preceding claims, wherein water and glycerol are fed as a gaseous mixture to the reactor.

5. The method according to any one of the preceding claims, wherein at least part of hydrogen gas, present in the effluent of the reactor, is separated from other gases in the effluent of the reactor and subsequently fed to the reactor once more.

6. The method according to any one of the preceding claims, wherein at least part of the effluent of the reactor, comprising acrolein, is condensed, and acrolein, present in the condensate, subsequently is separated, such as vaporized, from the condensate.

7. The method according to any one of the preceding claims, wherein the water and the glycerol are present in the mixed stream in a molar ratio of 20:1 to 5:1.

8. The method according to claim any one of the preceding claims, wherein the molar amount of hydrogen in the mixed stream is two times the molar amount of glycerol or less.

9. The method according to any one of the preceding claims, wherein the pressure over said catalyst is from 0.1 to 2 MPa, such as 0.1 to 1 MPa.

10. A method of producing acrylic acid from glycerol, comprising the steps of:
- dehydrating aqueous glycerol to acrolein in accordance with any one of the preceding claims;
- separating hydrogen from other gases present in the effluent of the reactor, in which glycerol is dehydrated to acrolein, by condensing water and acrolein;
- optionally feeding the separated hydrogen to the reactor once more;
- separating acrolein from the condensate, comprising water and acrolein, by vaporization of it;
- mixing the vaporized acrolein with oxygen; and
- passing the mixed gaseous stream, comprising acrolein and oxygen, over a catalyst, catalyzing the oxidation of acrolein to acrylic acid, at a temperature of 200 to 400°C, to obtain acrylic acid.

11. The method according to claim 10, wherein said catalyst, catalyzing the oxidation of acrolein to acrylic acid, is a catalyst comprising molybdenum and vanadium oxides and at least one further component selected from the group consisting of tungsten, copper, tin, antimony, niobium, nickel, cobalt, tellurium, bismuth, iron, lead, zinc, silicon, titanium, zirconium, alkaline metals, and oxides thereof.

12. Use of hydrogen to prevent formation of carbon deposits on an acidic catalyst, catalyzing the dehydration of glycerol to acrolein, in the dehydration of glycerol, in gas phase and in the presence of water, to acrolein.

13. The use according to claim 12, in a method according to any one of the claims 1 to 11.
